(19) Europäisches Patentamt
European Patent Office
Office européen des brevets

(11) Veröffentlichungsnummer: **0 563 731 B1**

(12) # EUROPÄISCHE PATENTSCHRIFT

(45) Veröffentlichungstag der Patentschrift: **15.11.95**

(51) Int. Cl.[6]: **A61K 31/765**, F02D 41/26

(21) Anmeldenummer: **93104655.1**

(22) Anmeldetag: **22.03.93**

(54) **Arzneimittel aus Polyhydroxymethylenderivaten, Verfahren zu deren Herstellung und Verwendung.**

(30) Priorität: **28.03.92 DE 4210220**

(43) Veröffentlichungstag der Anmeldung:
**06.10.93 Patentblatt 93/40**

(45) Bekanntmachung des Hinweises auf die Patenterteilung:
**15.11.95 Patentblatt 95/46**

(84) Benannte Vertragsstaaten:
**AT BE CH DE DK ES FR GB GR IE IT LI LU MC NL PT SE**

(56) Entgegenhaltungen:
**EP-A- 0 334 673**
**EP-A- 0 400 848**
**EP-A- 0 459 632**
**DE-A- 3 413 904**

(73) Patentinhaber: **HOECHST AKTIENGESELL-SCHAFT**

**D-65926 Frankfurt am Main (DE)**

(72) Erfinder: **Kesseler, Kurt, Dr.**
**Am kühlen Grund 3**
**W-6237 Liederbach (DE)**
Erfinder: **Schmidtberger, Rudolf**
**Salegrund 1**
**W-3550 Marburg (DE)**
Erfinder: **Müllner, Stefan, Dr.**
**Friedrich-Ebert-Strasse 43**
**W-6203 Hochheim (DE)**
Erfinder: **Granzer, Ernold, Dr. Dr.**
**Falkensteiner Strasse 24**
**W-6233 Kelkheim/Taunus (DE)**

**EP 0 563 731 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Arzneimittel enthaltend Polyhydroxymethylenderivate und ihre Verwendung zur Behandlung von Dyslipoproteinämien.

Gegenwärtig werden zur Behandlung von Dyslipoproteinämien verschiedene Strategien je nach Art und Schwere des Krankheitsbildes, der Patienten-Historie und der Einflüße anderer Risikofaktoren, angewandt. Durch die Entwicklung und Markteinführung der HMG-CoA-Reduktase-Inhibitoren ist es seit neuerer Zeit möglich, gezielt in die Cholesterinbiosynthese, insbesondere in die Cholesterinbiosynthese der Leber einzugreifen, um so den Plasmacholesterinspiegel wirksam zu senken. Die schon seit einiger Zeit auf dem Markt befindlichen Lipidsenker, wie z. B. Gallensäuresquestrantien (Colestyramin), Antioxidantien (Probucol), Fibrate (Bezafibrat, Clofibrat), Nikotinsäure und Derivate (Acipimox), Neomycin und Pflanzensterole (Sitosterol), Hormone und omega-3-Fettsäuren sind damit zwar nicht gänzlich unbedeutend geworden, besitzen aber seither nur noch Bedeutung für spezielle Indikationen.

Nachteilig bei den meisten genannten Lipidsenkern sind ihre doch zum Teil erheblichen Nebenwirkungen, so daß sich ihr Einsatz z. T. nur noch bei der Re-Infarktprophylaxe und bei ererbter Dyslipoproteinämie rechtfertigen läßt. Nach den Ergebnissen der großangelegten klinischen Studien der letzten Jahre, z. B. Framingham Study und Helsinki Heart Study, müßte man aber erheblich früher bei erhöhten Cholesterinwerten, insbesondere bei jungen Menschen und Menschen mittleren Alters, medikamentös eingreifen, um wirksam gegen die Todesursache Nr. 1 in der westlichen Welt, die Atherosklerose, vorzugehen. Dies führt jedoch unmittelbar zu Akzeptanzproblemen bei den Patienten, die wegen des fehlenden Gefühls krank zu sein, das Risiko nicht einsehen können und somit auch nicht bereit sind, Nebenwirkungen zu akzeptieren, wo sich simultan keine spürbare Wirkung einstellt. Andererseits bedeuten strenge diätitische Maßnahmen bei bescheidener Wirksamkeit oft weit erheblichere Einschränkungen für die Patienten als die Belastung mit bekannten Nebenwirkungen.

Der vorliegenden Erfindung liegt die Aufgabe zu Grunde, dem Wunsch der behandelnden Ärzte entsprechend, einen Lipidsenker zur Verfügung zu stellen, der

1. keine oder nur geringfügige Nebenwirkungen bei Langzeitanwendung zeigt,
2. sich durch eine hohe Palatibilität auszeichnet und in einer für den Patienten idealen Formulierung, z. B. Tablette, Kapsel, Drageè o. ä. zur Verfügung zu steht und
3. wirksam und effizient den Plasmacholesterinspiegel senkt.

Idealer Weise sollten dies nicht systemisch wirkende Arzneimittel sein, welche so beschaffen sein müssen, daß sie die Darmpassage in nicht resorbierbarer und nicht degradierbarer Art und Weise überstehen.

Zusätzlich muß, wie bei allen Arzneimitteln, eine toxikologische Unbedenklichkeit gegeben sein.

Die genannten Anforderungen werden überraschenderweise durch ein Arzneimittel erfüllt, das mindestens ein Polyhydroxymethylen mit aufgepfropften oxyethylierten Alkoholen und/oder oxyethylierten Carbonsäuren enthält.

Gegenstand der Erfindung ist daher ein Arzneimittel, gekennzeichnet durch einen wirksamen Gehalt eines Polyhydroxymethylens mit aufgepfropften oxyethylierten Alkoholen und/oder oxyethylierten Carbonsäuren.

Bei dem Polyhydroxymethylen mit aufgepfropften oxyethylierten Alkoholen und/oder oxyethylierten Carbonsäuren handelt es sich um ein Polymerisat, im wesentlichen enthaltend Einheiten (I) und/oder (II) als Bestandteile des Basispolymeren

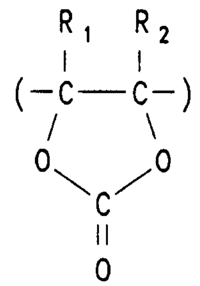

(I)

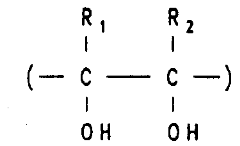 ,

(II)

worin $R_1$ und $R_2$ unabhängig voneinander Wasserstoff oder einen einwertigen Kohlenwasserstoffrest mit bis zu 8 Kohlenstoffatomen bedeuten und gegebenenfalls kleiner Mengen von weiteren Monomer-Einheiten, wobei das Basispolymer noch Einheiten kovalent gebunden hält, die sich ableiten von mindestens einer Verbindung der Formel (III)

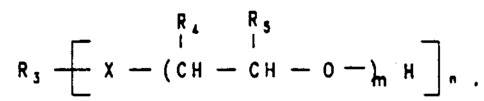

(III)

worin die Symbole $R_3$, X, $R_4$, $R_5$, m und n die folgende Bedeutung haben:

- $R_3$ einen Kohlenwasserstoffrest mit 4 bis 30 Kohlenstoff-Atomen, vorzugsweise einen Alkylrest mit 4 bis 30 Kohlenstoff-Atomen, vorzugsweise mit 12 bis 20 Kohlenstoff-Atomen oder einen Arylrest mit 6 bis 14 Kohlenstoff-Atomen, vorzugsweise mit 6 bis 10 Kohlenstoff-Atomen, vorzugsweise einen Phenylrest, wobei dieser Aryl(Phenyl)-rest gegebenenfalls mit einem, vorzugsweise 1 bis 3 Alkylresten mit 4 bis 30 Kohlenstoff-Atomen, vorzugsweise 12 bis 20 Kohlenstoff-Atomen, substituiert sein kann;
- X -O-, -NH- und/oder -COO-, vorzugsweise -COO- und/oder -O-;
- $R_4$ und $R_5$ unabhängig voneinander Wasserstoff oder einen einwertigen Kohlenwasserstoffrest, insbesondere einen Alkylrest, mit 1 bis 8 Kohlenstoff-Atomen, mit der Maßgabe, daß mindestens einer der Reste $R_4$ und $R_5$ Wasserstoff ist;
- m eine ganze Zahl von 1 bis 40, vorzugsweise 5 bis 35;
- n eine ganze Zahl von 1 bis 4, vorzugsweise 1 oder 2.

Das Basispolymere des Polymerisates besteht im wesentlichen, vorzugsweise zu mindestens 90 Gew.-%, aus den obigen Einheiten (I) und/oder (II) $R_1$ und $R_2$ bedeuten, vorzugsweise einen Alkylrest mit 1 bis 6 Kohlenstoffatomen und insbesondere einen solchen mit 1 bis 4 Kohlenstoffatomen. Beispiele hierfür sind: der Methyl-, Ethyl-, Isopropyl-, 2-Ethyl-hexyl-, n-Heptyl-, Cyclopentyl-, Phenyl-, Tolyl-, Benzyl- oder Xylylrest.

Besonders bevorzugt stehen die Reste $R_1$ und $R_2$ für Wasserstoff.

Nach einer weiteren bevorzugten Ausgestaltungsform der Erfindung beträgt die Menge an Einheiten (II) mehr als 50 Gew.-%, bezogen auf die Gesamtmenge von (I) und (II), d. h. die Cyclocarbonatgruppen von (I) sind mehrheitlich zu Hydroxygruppen verseift. Insbesondere beträgt die Menge an (II) mindestens 95 Gew.-% und besonders bevorzugt 100 Gew.-%.

Gegebenenfalls kann das Basispolymere noch geringe Mengen anderer Monomer-Einheiten enthalten, die sich von Monomeren ableiten, die mit Vinylencarbonat bzw. dessen Derivaten copolymerisierbar sind. Diese Monomeren können auch hydrophile oder vernetzende Gruppen aufweisen. Als Beispiele für derartige Monomere seien hier aufgeführt: Vinylpyrrolidon, (Meth)-Acrylsäurealkylester mit jeweils 2 bis 6 C-Atomen in der Alkylgruppe, Hydroxyalkylester der (Meth)Acrylsäure mit 2 bis 6 C-Atomen in der Alkylgruppe, N-Vinyl-N-alkylacetamid ($C_1$-$C_4$-Alkyl), Vinylacetat, Divinylether von Glykolen wie Ethylenglykoldivinylether, Butandioldivinylether, Hexandioldivinylether, N,N'-Alkylenbis(meth)acrylamide mit bis zu 12 C-Atomen,

vorzugsweise bis zu 6 C-Atomen enthaltenden geradkettigen oder verzweigten Alkylenresten wie N,N'-Methylenbisacrylamid, N,N'-Ethylenbisacrylamid, N,N'-Hexamethylenbisacrylamid, N,N'-Ethylidenbisacrylamid, Glyoxalbisacrylamid (1,2-Bisacrylamido-1,2-dihydroxyethan), Bisacrylamido-essigsäure, Ethylenglykol-bis-methacrylsäure-glykol-bis-methacrylsäureester, Butandiolbismethacrylsäureester, Triallylcyanurat, Trisacryloylperhydrotriazin, Divinylbenzol, Adipinsäuredivinylester, N,N'Divinylethylenharnstoff, N,N'-Divinylpropylenharnstoff, Ethyliden-bis-3-(N-vinylpyrrolidon), N,N'-Divinyldiimidazolyl(2,2') und 1,1'-Bis(3,3'-vinylbenzimidazolid-2-on)-1,4-butan, Vinylacrylat, Allylmethacrylat, Acryl- und Methacrylsäureester mit 5 bis 12 C-Atomen im Alkylrest, (Meth)Acrylnitril, Vinylester mit 4 bis 18 C-Atomen in dem Carbonsäurerest, wie Vinylbutyrat, Vinylstearat, und Vinylester verzweigter Carbonsäuren mit 10 bis 12 C-Atomen; weiterhin Vinylaromaten, wie Styrol oder $\alpha$-Methylstyrol.

Es können auch mehrere sich von diesen Monomeren ableitende Einheiten im Basispolymeren enthalten sein.

Die Menge dieser Monomer-Einheiten - falls vorhanden - übersteigt im allgemeinen nicht 15 Gew.-% und beträgt bevorzugt höchstens 10 Gew.-%, jeweils bezogen auf das gesamte Basispolymere.

Bevorzugt besteht das Basispolymere jedoch nur aus den Einheiten (I) und/oder (II).

Das Basispolymere enthält Einheiten, die sich von mindestens einer Verbindung der Formel (III) ableiten. Diese Einheiten können dabei auch von verschiedenen Verbindungen der Formel (III) stammen, d. h. es können bei der Herstellung der erfindungsgemäßen Polymerisate auch Gemische der verschiedenen Verbindungen gemäß Formel (III) zum Einsatz kommen.

Die Menge dieser Einheiten gemäß (III) beträgt im Regelfall bis zu 30 Gew.-%, vorzugsweise 1 bis 20 Gew.-%, und insbesondere 7 bis 15 Gew.-%, jeweils bezogen auf das Basispolymere.

In der obigen Formel (III) bedeuten die einzelnen Substituenten bevorzugt folgendes:

| | |
|---|---|
| $R_3$ | ein verzweigter, vorzugsweise aber unverzweigter Alkylrest mit 4 bis 30 Kohlenstoff-Atomen, vorzugsweise 12 bis 20 Kohlenstoff-Atomen. Beispielhaft seien genannt: Hexyl-, Heptyl-, Octyl-, Nonyl-, Decyl-, Undecyl-, Dodecyl-, Hexadecyl-, Octadecyl-, Eikosyl-. |
| X | -O-; -NH- und/oder -COO-; insbesondere -O-; |
| $R_4$ und $R_5$ | Wasserstoff oder Methyl, insbesondere Wasserstoff; |
| m | eine ganze Zahl von 5 bis 35; |
| n | 1 oder 2, insbesondere 1. |

Bevorzugte Vertreter der Formel (III) sind dementsprechend oxethylierte, einbasische, aliphatische Carbonsäuren (($C_4$-$C_{30}$)-Alkyl), wobei auch Gemische derartiger Säuren (z. B. Fettsäuren) in Frage kommen. Als besonders bevorzugte Vertreter sind hier oxethylierte einwertige, aliphatische Alkohole (($C_4$-$C_{30}$)-Alkyl) zu nennen, die bevorzugt primär sind. Auch hier sind Gemische derartiger Alkohole (z. B. Fettalkohole) verwendbar.

Mögliche Vertreter der Formel (III) sind beispielsweise auch oxethylierte, einwertige, insbesondere primäre Amine (X = -NH-), oxethylierte Hydroxycarbonsäuren (X = -O- und -COO-, n = 2), oxethylierte aromatische Carbonsäuren ($R_3$ = Phenyl) oder mehrwertige/mehrbasische Alkohole/Carbonsäuren ($n > 1$).

Bevorzugt sind demnach Polyhydroxymethylene, jeweils enthaltend Einheiten von oxethylierten, einbasischen, aliphatischen Carbonsäuren (($C_4$-$C_{30}$)-Alkyl) oder insbesondere oxethylierten einwertigen, aliphatischen Alkoholen (($C_4$-$C_{30}$)-Alkyl) in bevorzugten Mengen von 1 bis 20 Gew.-%, insbesondere von 7 bis 15 Gew.-%.

Das Basispolymere und die Einheiten, abgeleitet von den Verbindungen gemäß Formel (III) sind durch kovalente Bindungen miteinander verknüpft. Dabei sollte der überwiegende Teil der im Polymerisat eingebauten Einheiten, abgeleitet von den Verbindungen gemäß Formel (III) auf das Basispolymere aufgepfropft sein. Daneben ist auch der Einbau als Endgruppen denkbar. Bei größeren Zahlenwerten für m, d. h. bei längeren Polyetherresten kann es auch zu einer Pfropfung von Polyvinylencarbonatketten auf die alkoxylierten Verbindungen gemäß Formel (III) kommen.

Die Herstellung dieser Verbindungen wird in der DE-A-34 13 904 beschrieben.

Die Wirkung der beschriebenen Polyhydroxymethylenderivate beinhaltet die Adsorption von diätetischem Cholesterin und die sich daraus ergebende Resorptionshemmung. Diese Hemmung führt zu einer spürbaren Senkung des Serumcholesterinspiegels. Da das Medikament selbst nicht resorbierbar ist, ist auch nicht damit zu rechnen, daß Nebenwirkungen wie beim systemisch wirksamen Lipidsenker auftreten. Ein weiterer Vorteil der Polyhydroxymethylenderivate besteht in ihrer absoluten Geschmacks- und Geruchsneutralität und ihrer angenehmen Konsistenz, aufgrund derer zur Formulierung von Patienten-Compliance erhöhen den Zubereitungen keinerlei Zuschläge verwandt werden müssen.

Die vorgeschlagene Dosierung der erfindungsgemäß angewandten Polyhydroxymethylenderivate liegt nach den Daten der Tierversuchsergebnisse pro Tag bezogen auf 1 kg Körpergewicht im Bereich von 50 mg bis 5 g, vorzugsweise bei Dosen von 75 mg bis 1000 mg, besonders bevorzugt bei Dosen von 100 mg

bis 500 mg und ganz besonders bevorzugt bei Dosen von 125 mg bis 300 mg.

Ein weiterer überraschender Befund ist, daß eine Kombination der genannten Polyhydroxymethylenderivate mit anderen Gallensäuresequestrantien, wie z.B. Cholestyramin oder Colestipol oder insbesondere mit unvernetzten oder vernetzten alkylierten Polyethyleniminderivaten sowie mit anderen Lipidsenkern wie Fibraten, Probucol, Sitosterin, Nicotinsäure(derivaten) oder HMG-CoA-Reduktase-Inhibitoren möglich ist und die Verwendung dieser Kombination bei mindestens gleichbleibender oder sogar gesteigerter Wirkung eine Dosisverminderung bei den Polyhydroxymethylenderivaten zuläßt.

Gegenstand der Erfindung ist daher ebenso ein Arzneimittel, gekennzeichnet durch einen Gehalt an einer wirksamen Kombination aus zumindest einem Polyhydroxymethylen mit aufgepfropften oxyethylierten Alkoholen und/oder oxyethylierten Carbonsäuren der allgemeinen Formel (I) bis (III) und zumindest einem unvernetzten oder vernetzten alkylierten Polyethyleniminderivat, einem anderen Gallensäuresequestrans, z.B. Cholestyramin oder Colestipol oder einem anderen Lipidsenker, z.B. Fibrat, Probucol, Sitosterin, ein(e) Nicotinsäure(derivat) oder ein HMG-CoA-Reduktase-Inhibitor oder mit mehreren der obengenannten Polyethyleniminderivaten und/oder mehreren weiteren Gallensäuresequestrantien und/oder anderen Lipidsenkern.

Die Herstellung der erfindungsgemäß angewandten Polyethyleniminderivate ist in der DE-A-39 01 527 und den deutschen Patentanmeldungen P 41 31 507.3 und P 41 31 506.5 beschrieben. Die übrigen genannten Gallensäuresequestrantien bzw. Lipidsenker sind kommerziell erhältlich.

Die unvernetzten und vernetzten alkylierten Polyethylenimine sind dadurch charakterisiert, daß das Ausgangspolyethylenimin ein Molekulargewicht von 10 000 bis 10 000 000 aufweist und das Alkylierungsmittel die Formel (IV) besitzt

R - X,   (IV)

worin
X Chlor, Brom, Jod, $CH_3$-$SO_2$-O-oder

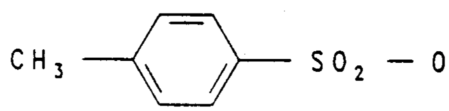

ist und
R ein geradkettiger, verzweigter oder cyclischer $C_1$ bis $C_{30}$-Alkylrest ist, der gegebenenfalls substituiert ist mit einem mono- oder bicyclischen gesättigten Kohlenwasserstoff mit 5 bis 10 Ringkohlenstoffatomen oder mit einem Phenylrest, und der im Falle eines vernetzten alkylierten Polyethylenimines als Vernetzungsmittel ein $\alpha,\omega$-Dihalogenalkan mit 2 bis 10 Kohlenstoffatomen oder ein höher funktionelles Halogenalkan mit 2 bis 10 Kohlenstoffatomen enthält.

Im Alkylierungsmittel, R-X ist X vorzugsweise Chlor oder Brom. Sofern R ein geradkettiger Rest ist, wird ein primärer Alkylrest bevorzugt.

Vorzugsweise sind die geradkettigen oder verzweigten $C_1$ bis $C_{30}$-Alkylreste mit den genannten Ringsystemen substituiert. Bevorzugt sind die Substituenten über Spacer mit 1 bis 4 $CH_2$-Gruppen mit dem Polyethylenimin verknüpft. Als monocyclischer gesättigter Substituent ist insbesondere der Cyclohexylrest geeignet. Ein geeigneter bicyclischer Kohlenwasserstoffrest ist z. B. Dekalin. Ein besonders geeignetes Alkylierungsmittel, dessen Alkylrest mit Phenyl substituiert ist, ist Benzylbromid. Als Alkylierungsmittel mit einem geradkettigen Alkylrest kommt vorzugsweise Butylchlorid in Betracht.

Handelt es sich um geradkettige oder verzweigte Reste R, so werden sowohl vernetzte als auch unvernetzte Polyethylenimine bevorzugt.

Steht R in Formel (IV) für einen cyclischen $C_5$ bis $C_{30}$-Alkylrest, so kann dieser monocyclisch, bicyclisch, tricyclisch oder polycyclisch sein, ggf. auch verbrückt.

Insgesamt bevorzugt sind unvernetzte cyclische Polyethylenimine, insbesondere solche, in denen R Cyclopentyl, Cyclohexyl, Cycloheptyl, Cyclooctyl, Cyclononyl, Cyclodecyl, einen bicyclischen Rest wie Decalyl, Hydrindanoyl, einen verbrückten Rest wie Norbornyl, oder einen polycyclischen Rest wie Cyclopentanoperhydrophenanthren und ein davon abgleitetes Ringsystem oder Derivat bedeutet.

Erfindungsgemäß werden vorzugsweise Polyethylenimine mit einem Molekulargewicht über 100 000 eingesetzt.

Die Alkylierung als solche kann in mehreren Teilschritten erfolgen. Dadurch besteht die Möglichkeit, verschiedene Substituenten am gleichen Polymeren zu fixieren.

Das Verhältnis des eingesetzten Alkylierungsmittels zu den Aminogruppen des Polyethylenimins beträgt 0,2:1 bis 5:1, vorzugsweise 0,5:1 bis 2:1.
Durch die Reaktion mit Alkylierungsmittel wird ein Teil der sekundären Aminogruppen der Kette in tertiäre und quartäre Strukturen überführt. Die Bildung von tertiären Aminogruppen wird bevorzugt.
Als Vernetzungsmittel eignen sich z. B. Di- und Trihalogenalkane, vorzugsweise $\alpha,\omega$-Dihalogenalkane wie z. B. 1,6-Dibromhexan und 1,10-Dibromdecan. Die Menge des Vernetzungsmittels beträgt vorzugsweise 2 bis 25 Mol-%, bezogen auf das eingesetzte Alkylierungsmittel.

Unter Verwendung bioinerter Trägerstoffe, wie Aktivkohle, Cellulose, Chitosan, Cyclodextrinen, Polyvinylpyrrolidon, Polyvinylalkohol oder Polyarylsäure(n)derivaten, z.B. ®Carbopol, sowie gegebenenfalls weiterer Zusatz- und/oder Hilfsstoffe werden die Arzneimittel in eine geeignete Darreichungsform, wie Tabletten, Kapseln, Dragees etc. gebracht.

Die erfindungsgemäßen Arzneimittel, enthaltend eine Kombination aus mindestens einem Polyhydroxymethylenderivat und mindestens einem weiteren Gallensäuresequestrans oder einem anderen Lipidsenker, besonders bevorzugt jedoch die Kombination aus Polyhydroxymethylenderivat(en) und Polyethyleniminderivat(en), zeigen besonders vorteilhafte Eigenschaften, insbesondere im Vergleich mit Arzneimitteln, ausschließlich enthaltend Polyhydroxymethylenderivate. So kann die Dosierung der Polyhydroxymethylenderivate in der kombinierten Anwendung bei gleichbleibender oder verbesserten Wirksamkeit gegenüber der alleinigen Anwendung reduziert werden.

Die vorgeschlagenen Dosierungen für die Bestandteile des vorstehend beschriebenen, erfindungsgemäßen Arzneimittels liegen nach den Daten von Tierversuchsergebnissen für die Polyhydroxymethylenderivate pro Tag bezogen auf 1 kg Körpergewicht im Bereich von 50 mg bis 5 g, vorzugsweise bei 75 mg bis 1 000 mg, besonders bevorzugt bei 100 mg bis 500 mg und ganz besonders bevorzugt bei 125 mg bis 300 mg und für die weiteren Gallensäuresequestrantien, insbesondere die Polyethyleniminderivate, pro Tag bezogen auf 1 kg Körpergewicht im Bereich von 10 mg bis 10 g, vorzugsweise von 25 mg bis 5 g, besonders bevorzugt von 40 mg bis 500 mg und ganz besonders bevorzugt bei 50 mg.

Die therapeutische Wirksamkeit des erfindungsgemäßen Arzneimittels wird anhand der Ergebnisse eines Tierversuchs an Kaninchen demonstriert.

Tierversuch:

Wirkung von Polyhydroxymethylenderivaten auf die Serumcholesterinkonzentration hypercholesterinämischer NZW-Kaninchen:

Methodik:

Bei männlichen NZW-Kaninchen mit einem durchschnittlichen Ausgangswert des Serumcholesterins von 25 mg/dl wurde durch eine zweiwöchige Gabe eines mit 0,2 % Cholesterin angereicherten Futters eine mäßige bis starke Hypercholesterinämie erzeugt. Es wurden drei Gruppen á 6 Tieren so zusammengestellt, daß deren mittlerer (erhöhter) Serum-Cholesteringehalt etwa 61 bis 69 mg/dl betrug. Unter den 6 Tieren waren jeweils 5 mit einer mäßigen Hypercholesterinämie enthalten (Werte zwischen 30 und 51 mg/dl) und 1 Tier mit einem stark erhöhten Serumcho-lesterinwert. Bei weiterem Angebot des mit 0,2 % Cholesterin angereicherten Kaninchenfutters erhielten sie oral per Schlundsonde Polyhydroxymethylen-L (PHM-L; Hersteller: Riedel de Haen, Artikelnummer 39 350) die Kontrolle lediglich das Vehikel 1 % ®Tylose MH 300 (Methylhydroxyethylcellulose; Hersteller: HOECHST AG).

Die Dosierungen betrugen 125 bzw. 500 mg/kg Körpergewicht/Tag PHM-L, einmal täglich verabfolgt. Insgesamt wurden 10 Applikationen verabreicht, und auch in der sich unmittelbaren anschließenden Auslaßphase (keine weitere Verabreichung von PHM-L oder Tylose®, jedoch weiterhin Gabe von Cholesterindiätfutter) wurde das Verhalten des Serumcholesterins verfolgt. Die Daten sind in den Tabellen 1a, b dargestellt. Neben den Absolutwerten (mg/dl Cholesterin) sind für die 2 Gruppen der mit PHM-L behandelten Tiere die mittleren prozentualen Veränderungen des Serumcholesterins gegenüber der Kontrollgruppe (1 % ®Tylose MH 300) angegeben. Es kann somit beurteilt werden, welchen Effekt die Behandlung mit PHM-L dosis- und zeitabhängig hat und wie sich die Seriumcholesterinkonzentration nach dem Absetzen der PHM-L-Behandlung, während der Auslaßphase weiterentwickelt.

Ergebnis:

Mit Dosierungen von 125 oder 500 mg/kg/Tag ist man in der Lage, die bestehende Hypercholesterinämie der Kaninchen zu reduzieren und dies auch anhaltend während der bis zur 32tägigen Auslaßphase. In einem analogen früheren Versuch war die Dosierung von 250 mg/kg/Tag ebenfalls antihypercholesterinämisch wirksam.

| VERSUCHSPLAN | |
|---|---|
| Gesamtversuchsdauer: | 58 Tage |
| Umstellung auf Cholesterindiätfutter: | 1. Tag |
| Erste PHM-L-Applikation: | 16. Tag |
| Letzte PHM-L-Applikation: | 26. Tag |
| Letzte Probennahme/Versuchsende: | 58. Tag |
| Tierart: | weiße männliche Neuseeländer-Kaninchen |
| Stamm: | NZB |
| Tierzahl: | 18 (3 x 6) |
| Applikationsart: | per os (Magensonde) |
| Vehikel: | 1 % ®Tylose MH 300 |
| Zahl der Applikationen: | 10 |
| Futterart: | 0,2 %ige Cholesterindiät basierend auf handelsüblichem Futtermehl |

Tabelle 1a:

| | | Serumcholesterin [mg/dl] | | | | | | |
|---|---|---|---|---|---|---|---|---|
| Präparat (Dosis) | Tier Nr. | Ausgangswerte vor Applikationsbeginn des Präparates | | Werte während der Applikation des Präparates | | Werte nach Absetzen des Präparates | | |
| | | 1. | 11. Diättag | 3. | 8. Applikationstag | 4. | 12. | 32. Auslaßtag |
| Kontrolle Tylose MH 300 (1 %ig) | 1 | 26 | 32 | 34 | 34 | 39 | 27 | 80 |
| | 2 | 24 | 32 | 48 | 43 | 38 | 32 | 168 |
| | 3 | 24 | 37 | 47 | 61 | 75 | 94 | - |
| | 4 | 26 | 40 | 43 | 47 | 28 | 25 | 104 |
| | 5 | 27 | 51 | 67 | 83 | 89 | 75 | 123 |
| | 6 | 36 | 221 | 350 | 364 | 464 | 406 | 601 |
| X | | 27 | 69 | 98 | 105 | 122 | 110 | 215 |
| S | | 4,5 | 74,8 | 124 | 128 | 169 | 148 | 218 |
| PHM-L (125 mg/kg/Tag) | 7 | 21 | 36 | 34 | 47 | 65 | 34 | 105 |
| | 8 | 20 | 30 | 21 | 18 | 20 | 20 | - |
| | 9 | 20 | 31 | 44 | 48 | 55 | 46 | 84 |
| | 10 | 27 | 42 | 28 | 35 | 35 | 30 | 66 |
| | 11 | 21 | 50 | 56 | 72 | 64 | 66 | 177 |
| | 12 | 26 | 203 | 247 | 270 | 337 | 361 | 380 |
| X | | 23 | 65 | 72 | 82 | 96 | 93 | 162 |
| S | | 3,2 | 68 | 87 | 94 | 119 | 132 | 129 |
| % von Kontrolle | | - | - | -27 % | -22 % | -21 % | -15 % | -25 % |

X = arithmetisches Mittel S = Standardabweichung

Tabelle 1b:

| Präparat (Dosis) | Tier Nr. | Serumcholesterin [mg/dl] | | | | | | |
|---|---|---|---|---|---|---|---|---|
| | | Ausgangswerte vor Applikationsbeginn des Präparates | | Werte während der Applikation des Präparates | | Werte nach Absetzen des Präparates | | |
| | | 1. | 11. Diättag | 3. | 8. Applikationstag | 4. | 12. | 32. Auslaßtag |
| PHM-L (500 mg/kg/Tag) | 13 | 28 | 38 | 28 | 32 | 39 | 30 | 57 |
| | 14 | 24 | 30 | 32 | 31 | 28 | 22 | 54 |
| | 15 | 20 | 31 | 41 | 65 | 57 | 67 | 90 |
| | 16 | 28 | 43 | 42 | 41 | 58 | 64 | 77 |
| | 17 | 28 | 51 | 96 | 71 | 38 | 47 | 100 |
| | 18 | 26 | 174 | 152 | 178 | 170 | 165 | 333 |
| X | | 26 | 61 | 65 | 70 | 65 | 66 | 119 |
| S | | 3,9 | 56 | 49 | 56 | 53 | 52 | 107 |
| % von Kontrolle | | - | - | -34 % | -33 % | -47 % | -40 % | -45 % |

X = arithmetisches Mittel S = Standardabweichung

## Patentansprüche

**1.** Arzneimittel, enthaltend als Wirkstoff ein Polymerisat, dadurch gekennzeichnet, daß das Polymerisat im wesentlichen Einheiten (I) und/oder (II) als Bestandteile des Basispolymeren enthält

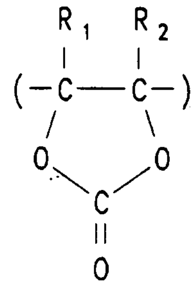

(I)

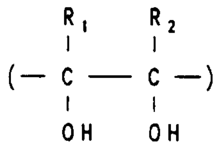

,

(II)

worin $R_1$ und $R_2$ unabhängig voneinander Wasserstoff oder einen einwertigen Kohlenwasserstoffrest mit bis zu 8 Kohlenstoffatomen bedeuten, und gegebenenfalls kleine Mengen von weiteren Monomer-Einheiten, wobei das Basispolymer noch Einheiten kovalent gebunden enthält, die sich ableiten von mindestens einer Verbindung der Formel (III)

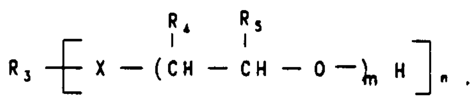

(III)

worin die Symbole $R_3$, X, $R_4$, $R_5$, m und n die folgende Bedeutung haben:

| | |
|---|---|
| $R_3$ | einen Kohlenwasserstoffrest mit 4 bis 30 Kohlenstoff-Atomen; |
| X | -O-; -NH- und/oder -COO-; |
| $R_4$ und $R_5$ | unabhängig voneinander Wasserstoff oder einen einwertigen Kohlenwasserstoffrest mit 1 bis 8 Kohlenstoff-Atomen, mit der Maßgabe, daß mindestens einer der Reste $R_4$ und $R_5$ Wasserstoff ist; |
| m | eine ganze Zahl von 1 bis 40; |
| n | 1, 2, 3 oder 4; |

**2.** Arzneimittel nach Anspruch 1, dadurch gekennzeichnet, daß das Basispolymere mindestens 95 Gew.-% Einheiten der Formel (II), bezogen auf die Gesamtmenge an Einheiten (I) und (II), enthält.

**3.** Arzneimittel nach Anspruch 1 und/oder 2, dadurch gekennzeichnet, daß die Menge an Einheiten gemäß Formel (III) 1 bis 20 Gew.-%, bezogen auf das Basispolymere, beträgt.

**4.** Arzneimittel nach einem oder mehreren der Ansprüche 1 bis 3, dadurch gekennzeichnet, daß $R_1$ und $R_2$ in Formel (I) und Formel (II) für Wasserstoff stehen.

**5.** Arzneimittel nach einem oder mehreren der Ansprüche 1 bis 4, dadurch gekennzeichnet, daß $R_3$ für einen Alkylrest mit 4 bis 30 Kohlenstoffatomen, X für -O- und/oder -COO-, $R_4$ und $R_5$ für Wasserstoff, m für 5 bis 35 und n für 1 oder 2 stehen.

**6.** Arzneimittel nach einem oder mehreren der Ansprüche 1 bis 5, dadurch gekennzeichnet, daß $R_3$ für einen Alkylrest mit 12 - 20 Kohlenstoffatomen und X für -O- und/oder -COO- stehen.

**7.** Arzneimittel nach Anspruch 6, dadurch gekennzeichnet, daß X für -O- steht.

**8.** Arzneimittel nach einem oder mehreren der Ansprüche 1 bis 7, dadurch gekennzeichnet, daß es ein Gemisch aus mindestens einem Polymerisat, enthaltend Einheiten gemäß den Formeln (I) und/oder (II)

und mindestens einer Einheit, abgeleitet von einer Verbindung der Formel (III) und mindestens einem vernetzten oder unvernetzten alkylierten Polyethylenimin, wobei das Ausgangspolyethylenimin eine Molmasse von 10 000 bis 10 000 000 aufweist und das Alkylierungsmittel der Formel (IV) entspricht

R-X, (IV)

worin
X Chlor, Brom, Jod, $CH_3$-$SO_2$-O- oder

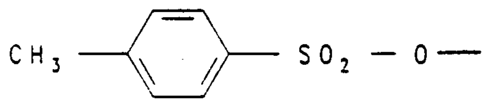

ist und
R ein geradkettiger, verzweigter oder cyclischer $C_1$ bis $C_{30}$-Alkylrest ist, der gegebenenfalls substituiert ist mit einem mono- oder bicyclischen gesättigten Kohlenwasserstoff mit 5 bis 10 Ringkohlenstoffatomen oder mit einem Phenylrest und der im Falle eines vernetzten alkylierten Polyethylenimins als Vernetzungsmittel ein $\alpha,\omega$-Dihalogenalkan mit 2 bis 10 Kohlenstoffatomen oder ein höher funktionelles Halogenalkan mit 2 bis 10 Kohlenstoffatomen enthält.

**9.** Arzneimittel nach Anspruch 8, dadurch gekennzeichnet, daß R in Formel IV ein ggf. verbrückter Cycloalkylrest mit 5 bis 30 Kohlenstoffatomen ist, der monocyclisch, bicyclisch, tricyclisch oder polycyclisch ist.

**10.** Arzneimittel nach den Ansprüchen 8 oder 9, dadurch gekennzeichnet, daß R in Formel (IV) Cyclopentyl, Cyclohexyl, Cycloheptyl, Cyclooctyl, Cyclononyl, Cyclodecanyl, einen bicyclischen Rest wie Decalyl, Hydrindanoyl, einen verbrückten Rest wie Norbornyl, oder einen polycyclischen Rest wie Cyclopentanoperhydrophenanthren oder ein davon abgeleitetes Ringsystem oder Derivat bedeutet.

**11.** Arzneimittel nach einem oder mehreren der Ansprüche 1 bis 8, dadurch gekennzeichnet, daß sie Polyethylenimine mit einem Molekulargewicht von 100 000 bis 10 000 000 enthalten.

**12.** Arzneimittel nach einem oder mehreren der Ansprüche 1 bis 8, dadurch gekennzeichnet, daß es zusätzlich ein weiteres ein Gallensäuresequestrans und/oder einen anderen Lipidsenker enthält.

**13.** Arzneimittel nach einem oder mehreren der Ansprüche 1 bis 12, dadurch gekennzeichnet, daß es bioinerte Trägerstoffe wie Aktivkohle, Cellulose, Chitosan, Cyclodextrine, Polyvinylpyrrolidon, Polyvinylalkohol oder Polyacrylsäure(n)derivate, sowie gegebenenfalls weitere Zusatz- und/oder Hilfsstoffe enthält.

**14.** Arzneimittel nach einem oder mehreren der Ansprüche 1 bis 13 zur Anwendung in einem Verfahren zur Senkung eines erhöhten Plasmacholesterinspiegels.

**15.** Verfahren zur Herstellung eines Arzneimittels nach einem oder mehreren der Ansprüche 1 bis 11, dadurch gekennzeichnet, daß das Polymerisat, enthaltend Einheiten gemäß den Formeln (I) und/oder (II) und mindestens einer Einheit, abgeleitet von einer Verbindung der Formel (III) sowie gegebenenfalls ein Polyethyleniminderivat mit einem physiologisch annehmbaren Träger und gegebenenfalls weiteren Zusatz- und/oder Hilfsstoffen in eine geeignete Darreichungsform gebracht wird.

**Claims**

**1.** A pharmaceutical containing a polymer as active ingredient, wherein the polymer essentially contains units (I) and/or (II) as constituents of the base polymer

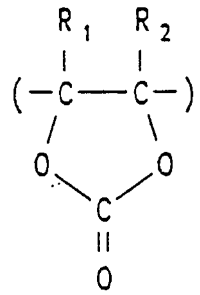

(I)

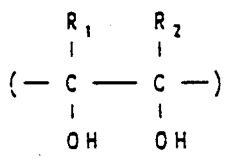

(II)

in which $R_1$ and $R_2$ independently of one another are hydrogen or a monovalent hydrocarbon radical having up to 8 carbon atoms,
and optionally small amounts of further monomer units, the base polymer additionally containing units covalently bound which are derived from at least one compound of the formula (III)

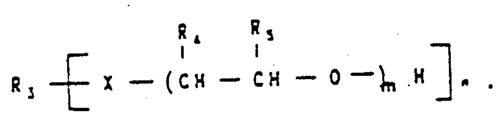

(III)

in which the symbols $R_3$, X, $R_4$, $R_5$, m and n have the following meaning:

| | |
|---|---|
| $R_3$ | is a hydrocarbon radical having 4 to 30 carbon atoms; |
| X | is -O-, -NH- and/or -COO-; |
| $R_4$ and $R_5$ | independently of one another are hydrogen or a monovalent hydrocarbon radical having 1 to 8 carbon atoms, with the proviso that at least one of the radicals $R_4$ and $R_5$ is hydrogen; |
| m | is an integer from 1 to 40; |
| n | is 1, 2, 3 or 4. |

**2.** A pharmaceutical as claimed in claim 1, wherein the base polymer contains at least 95% by weight of units of the formula (II), relative to the total amount of units (I) and (II).

**3.** A pharmaceutical as claimed in claim 1 and/or 2, wherein the amount of units according to formula (III) is 1 to 20% by weight, relative to the base polymer.

**4.** A pharmaceutical as claimed in one or more of claims 1 to 3, wherein $R_1$ and $R_2$ in formula (I) and formula (II) are hydrogen.

**5.** A pharmaceutical as claimed in one or more of claims 1 to 4, wherein $R_3$ is an alkyl radical having 4 to 30 carbon atoms, X is -O- and/or -COO-, $R_4$ and $R_5$ are hydrogen, m is 5 to 35 and n is 1 or 2.

**6.** A pharmaceutical as claimed in one or more of claims 1 to 5, wherein $R_3$ is an alkyl radical having 12-20 carbon atoms and X is -O- and/or -COO-.

**7.** A pharmaceutical as claimed in claim 6, wherein X is -O-.

**8.** A pharmaceutical as claimed in one or more of claims 1 to 7, which contains a mixture of at least one polymer containing units according to the formulae (I) and/or (II) and at least one unit derived from a compound of the formula (III) and at least one crosslinked or uncrosslinked alkylated polyethyleneimine,

the starting polyethyleneimine having a molar mass of 10,000 to 10,000,000 and the alkylating agent corresponding to the formula (IV)

R - X (IV)

in which
X is chlorine, bromine, iodine, $CH_3$-$SO_2$-O or

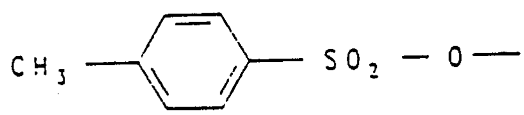

and
R is a straight-chain, branched or cyclic $C_1$ to $C_{30}$-alkyl radical, which is optionally substituted by a mono- or bicyclic saturated hydrocarbon having 5 to 10 ring carbon atoms or by a phenyl radical, and which in the case of a crosslinked alkylated polyethyleneimine contains as a crosslinking agent an $\alpha,\omega$-dihaloalkane having 2 to 10 carbon atoms or a more highly functionalized haloalkane having 2 to 10 carbon atoms.

**9.** A pharmaceutical as claimed in claim 8, wherein R in formula IV is an optionally bridged cycloalkyl radical having 5 to 30 carbon atoms, which is monocyclic, bicyclic, tricyclic or polycyclic.

**10.** A pharmaceutical as claimed in either of claims 8 and 9, wherein R in formula (IV) is cyclopentyl, cyclohexyl, cycloheptyl, cyclooctyl, cyclononyl, cyclodecyl, a bicyclic radical such as decalyl, hydrindanoyl, a bridged radical such as norbornyl, or a polycyclic radical such as cyclopentanoperhydrophenanthrene or a ring system or derivative derived therefrom.

**11.** A pharmaceutical according to one or more of claims 1 to 8, which contains polyethyleneimines having a molecular weight of 100,000 to 10,000,000.

**12.** A pharmaceutical as claimed in one or more of claims 1 to 8, which additionally contains a further bile acid sequestrant and/or another hypolipidemic agent.

**13.** A pharmaceutical according to one or more of claims 1 to 12, which contains bio-inert excipients such as active carbon, cellulose, chitosan, cyclodextrins, polyvinylpyrrolidone, polyvinyl alcohol or polyacrylic acid(s) derivatives, and optionally further additives and/or auxiliaries.

**14.** A pharmaceutical according to one or more of claims 1 to 13 for use in a process for decreasing an increased plasma cholesterol level.

**15.** A process for the production of a pharmaceutical as claimed in one or more of claims 1 to 11, which comprises bringing the polymer containing units according to the formulae (I) and/or (II) and at least one unit derived from a compound of the formula (III) and optionally a polyethyleneimine derivative into a suitable form for administration using a physiologically acceptable excipient and, if appropriate, further additives and/or auxiliaries.

**Revendications**

**1.** Médicament contenant en tant que substance active un polymère, caractérisé en ce que le polymère contient essentiellement, comme composants du polymère de base, des motifs (I) et/ou (II)

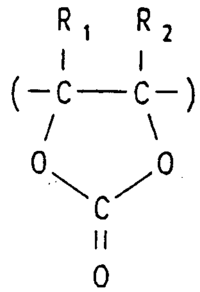

(I)

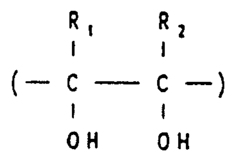

, (II)

dans lesquels $R_1$ et $R_2$ représentent, indépendamment l'un de l'autre, un atome d'hydrogène ou un radical hydrocarboné monovalent ayant jusqu'à 8 atomes de carbone, et éventuellement de petites quantités d'autres motifs monomères, le polymère de base contenant encore des motifs liés par covalence, qui dérivent d'au moins un composé de formule (III)

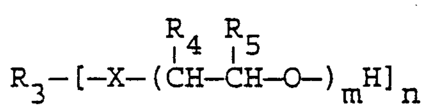

(III)

dans laquelle les symboles $R_3$, X, $R_4$, $R_5$, m et n ont les significations suivantes:

| | |
|---|---|
| $R_3$ | représente un radical hydrocarboné ayant de 4 à 30 atomes de carbone; |
| X | représente -O-, -NH- et/ou -COO-; |
| $R_4$ et $R_5$ | représentent, indépendamment l'un de l'autre, un atome d'hydrogène ou un radical hydrocarboné monovalent ayant de 1 à 8 atomes de carbone, étant entendu qu'au moins l'un des radicaux $R_4$ et $R_5$ est un atome d'hydrogène; |
| m | est un nombre entier allant de 1 à 40; |
| n | est 1, 2, 3 ou 4. |

**2.** Médicament selon la revendication 1, caractérisé en ce que le polymère de base contient au moins 95 % en poids de motifs de formule (II), par rapport à la quantité totale des motifs (I) et (II).

**3.** Médicament selon la revendication 1 et/ou la revendication 2, caractérisé en ce que la quantité de motifs de formule (III) va de 1 à 20 % en poids, par rapport au polymère de base.

**4.** Médicament selon une ou plusieurs des revendications 1 à 3, caractérisé en ce que, dans la formule (I) et la formule (II), $R_1$ et $R_2$ représentent des atomes d'hydrogène.

**5.** Médicament selon une ou plusieurs des revendications 1 à 4, caractérisé en ce que $R_3$ représente un radical alkyle ayant de 4 à 30 atomes de carbone, X représente -O- et/ou -COO-, $R_4$ et $R_5$ représentent des atomes d'hydrogène, m va de 5 à 35 et n représente 1 ou 2.

**6.** Médicament selon une ou plusieurs des revendications 1 à 5, caractérisé en ce que $R_3$ représente un radical alkyle ayant de 12 à 20 atomes de carbone et X représente -O- et/ou -COO-.

**7.** Médicament selon la revendication 6, caractérisé en ce que X représente -O-.

**8.** Médicament selon une ou plusieurs des revendications 1 à 7, caractérisé en ce qu'il contient un mélange d'au moins un polymère contenant des motifs de formule (I) et/ou de formule (II), et au moins un motif dérivé d'un composé de formule (III) et au moins une polyéthylène-imine alkylée, réticulée ou

non réticulée, la polyéthylène-imine de départ ayant une masse moléculaire de 10 000 à 10 000 000, et l'agent d'alkylation correspondant à la formule (IV)

R-X (IV)

dans laquelle
X est un atome de chlore, de brome ou d'iode ou le groupe $CH_3$-$SO_2$-O- ou

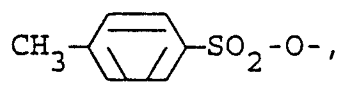

et
R est un radical alkyle en $C_1$-$C_{30}$ à chaîne droite, ramifié ou cyclique, qui est éventuellement substitué par un radical hydrocarboné saturé mono- ou bicyclique ayant de 5 à 10 atomes de carbone formant le ou les cycles ou par un groupe phényle et, dans le cas d'une polyéthylène-imine alkylée réticulée, en tant qu'agent de réticulation, un $\alpha,\omega$-dihalogénoalcane ayant de 2 à 10 atomes de carbone ou un halogénoalcane à plus haute fonctionnalité ayant de 2 à 10 atomes de carbone.

**9.** Médicament selon la revendication 8, caractérisé en ce que R dans la formule IV est un radical cycloalkyle éventuellement ponté, ayant de 5 à 30 atomes de carbone, qui est monocyclique, bicyclique, tricyclique ou polycyclique.

**10.** Médicament selon la revendication 8 ou 9, caractérisé en ce que R dans la formule (IV) représente le radical cyclopentyle, cyclohexyle, cycloheptyle, cyclooctyle, cyclononyle, cyclodécanyle, un radical bicyclique tel que le radical décalyle, hydrindanoyle, un radical ponté tel que le radical norbornyle, ou un radical polycyclique tel que le cyclopentanoperhydrophénanthrène ou un dérivé ou système cyclique en dérivant.

**11.** Médicament selon une ou plusieurs des revendications 1 à 8, caractérisé en ce qu'il contient des polyéthylène-imines ayant une masse moléculaire de 100 000 à 10 000 000.

**12.** Médicament selon une ou plusieurs des revendications 1 à 8, caractérisé en ce qu'il contient en outre un séquestrant d'acide biliaire et/ou un autre agent hypolipidémiant.

**13.** Médicament selon une ou plusieurs des revendications 1 à 12, caractérisé en ce qu'il contient des véhicules biologiquement inertes tels que le charbon actif, la cellulose, le chitosane, des cyclodextrines, la polyvinylpyrrolidone, le poly(alcool vinylique) ou des dérivés de poly(acide acrylique)(s), ainsi qu'éventuellement d'autres additifs et/ou adjuvants.

**14.** Médicament selon une ou plusieurs des revendications 1 à 13, pour utilisation dans un procédé pour l'abaissement d'un taux élevé de cholestérol plasmatique.

**15.** Procédé pour la fabrication d'un médicament selon une ou plusieurs des revendications 1 à 11, caractérisé en ce que l'on met sous une forme d'administration appropriée le polymère contenant des motifs de formule (I) et/ou de formule (II) et au moins un motif dérivé d'un composé de formule (III), ainsi qu'éventuellement un dérivé de polyéthylène-imine, avec un véhicule physiologiquement acceptable et éventuellement d'autres additifs et/ou adjuvants.